# EUROPEAN PATENT APPLICATION

(11) **EP 1 062 931 A1**
(43) Date of publication of application: **27.12.2000**
(21) Application number: 99112063.5
(22) Date of filing: 22.06.1999
(51) Int. Cl.: A61F 13/56, A61F 13/58, A61F 13/15

(54) **Adhesive and mechanical fastener systems for disposing absorbent articles**

(71) Applicant: SCA Hygiene Products AB, 405 03 Göteborg (SE)
(72) Inventor: Arezki, Salem, F-59250 Halluin (FR)
(74) Representative: Harrison, Michael Charles

(57) **Abstract**

Absorbent product (1) comprising a back sheet (4), a top sheet (2) and an attachment means (5, 8, 10; 6, 7, 9). The attachment means has one attachment part (5, 6) of a hook and loop fastening means and is positioned on a first strip (7). The strip (7) is attached on one side of the absorbent product at an edge (15) thereof such that the attachment part (5, 6) lies outwardly of an edge (15). Additionally there is an adhesive reclosure means, having a second material strip (12) coated with adhesive (11), the strip (12) overlies the edge (15) of one of the sheets (2, 4) of the absorbent article and a strip of release material (13) is interposed between the sheet (2) and the adhesive (11). In this way, premature exposure of the reclosure adhesive (11) is provided.

## Description

### Field of the invention:

The present invention relates to an absorbent product intended for the absorption of bodily exudates. In particular, the invention relates to diapers or other absorbent garments for child or adult use having reclosure means thereon in addition to releasable attachment means for fastening the garment around the user. Even more particularly, the invention relates to absorbent products which are disposable after use (i.e. of the type having materials which are not intended to be washed and used several times).

### Background to the invention:

Typically, absorbent products of the above-mentioned type have an absorbent core surrounded by an envelope comprising a fluid-impermeable sheet or layer (commonly referred to as a back sheet) and a fluid-permeable sheet or layer (commonly referred to as a liner, top sheet or bodyside layer). In order to provide fastening of the absorbent product around a user's waist, the absorbent product has attachment means which are typically either of an adhesive type such as pressure sensitive adhesive, or of a releasable mechanical engagement type often known generally as a hook and loop type attachment means (such as the type sold under the name Velcro®). Such means and their method of attachment are well known in the art.

A problem which presents itself with such articles is however the problem of how to dispose of the products after use in a suitably compact form. In particular this compact form is one which is often used to conceal and contain bodily exudate therein. To this end, it is known to roll up or fold up such absorbent products in order that the product obtains the desired compact form. It is also known that means can be provided for maintaining the product in this rolled-up or folded-up condition when disposing of it in a waste receptacle or the like, or whilst carrying it in a bag for later disposal.

However, when hook and loop type fastening means are used as the waist fastening members, the rolling-up of the product (which is performed by starting at one end) means that one of the corresponding hook or loop parts might become obscured inside the rolled-up product. Since the remaining hook or loop part (which is on the last part to be folded up) cannot however adhere to the back sheet material of the rolled up product, nor to the respective attachment part on the other side of the garment, an additional means of maintaining the product in its rolled up condition is required. Such additional means are here referred to as reclosure means.

The general manner of rolling up such products and maintaining them is described for example in EP-A-0 321 234 and thus no further description of said procedure is required.

Several solutions for reclosure means have been proposed, also in EP-A-0 321 234, which include such solutions as extra loop attachment material on the hook attachment strips, or extra adhesive means to act as a reclosure means. In one embodiment of EP-A-0 321 234, an adhesive is added on one of the two surfaces of the material strip which carries a hook attachment part of a hook and loop attachment means. The adhesive of the reclosure means is covered by a strip of release material, referred to as a release liner, which can be pivoted upwards at one end so as to expose the adhesive. In this condition the adhesive can then be adhered to the back sheet of the material.

Further solutions using adhesive reclosure means are known from WO-A-92/04001 in which an adhesive is present on two surfaces of the material strip which carries the hook part of the releasable attachment means. In that document an upper surface adhesive means is disclosed which is constantly exposed to external influence and therefore can be soiled. This soiling impairs its function. Thus a second adhesive means is provided on the material strip, which adhesive can be accessed by peeling up a part of a covering layer of the strip to reveal the underlying adhesive.

Such solutions are however disadvantageous in many respects since the normal handling of the hook tabs when attaching them to the corresponding loop attachment member on the front of the absorbent product can involve a great deal of manipulation and flexing of the material strips and sometimes even stretching of the strips. This is in particular the case when several refastening operations are required. This can result in premature peeling apart and exposure of the reclosure means adhesive, which is clearly undesirable. The peelable part of the strip which covers the adhesive may also crinkle up, making it difficult to re-attach properly.

Although the adhesive force between the peelable strip and the underlying adhesive could be increased to help prevent premature peeling separation, this of course makes the peeling operation itself particularly difficult when the user actually does wish to use the reclosure means, in particular since the amount of material which can be grasped to effect peeling is relatively small.

The present invention has the object of providing a solution to the aforementioned problem.

Further problems which can be solved by this invention with respect to known absorbent products will become apparent from the following description.

### Summary of the invention:

The aforementioned problem is solved by an absorbent product having the features defined in claim 1. Preferred features of the invention are defined in the dependent claims.

It should be understood that the term "hook and loop attachment means", as used herein, is intended to mean a mechanical engagement means consisting of two sets of cooperating engaging elements and is not limited to means which only have the form of an engaging hook and loop. For example, mushroom-shaped heads of the male engagement members are also intended to be encompassed by the term "hook".

The absorbent product of the invention is particularly advantageous since the reclosure adhesive means, when not required for use, are located on a part of the absorbent product remote from the mechanical engagement means. In particular, the adhesive overlies a portion of the top sheet and back sheet at an edge portion of the product and is thus located on a part of the product which is not normally subject to large amounts of bending or stretching during fitting. Thus, the strip of material on which the releasable attachment part is located is able to flex more easily and without effecting the reclosure attachment.

Due to the arrangement of the product of the present invention, the further advantage is obtained that the releasable attachment means (e.g. a hook part) can be spaced away from the adhesive reclosure tabs which means that when a user handles the attachment means during fastening, no oil, cream, or dirt which is on the user's hands need come into contact with the outer surface of the second strip covering the adhesive reclosure. This leaves the second strip more readily graspable when it is desired to peel it away from the underlying material layer(s). This is in stark contrast to prior art solutions where the releasable attachment means must either be on top of, or directly adjacent, the reclosure means.

### Brief description of the drawings:

The invention will now be explained in more detail with reference to certain non-limiting embodiments thereof and with the aid of the accompanying drawings, in which:
- Fig. 1: is a plan view of an absorbent product according to the invention, in the form of a diaper, placed with the top sheet thereof uppermost,
- Fig. 2: is a sectional view along line II-II of the attachment means and reclosure means combination at the upper right corner in Fig. 1, whilst the reclosure means in a closed condition.
- Fig. 3: is a sectional view corresponding to that shown in Fig. 2, but with the reclosure means open and ready for use.
- Fig. 4: shows a further embodiment of the invention in a sectional view similar to that shown in Fig. 3.
- Fig. 5: shows a further embodiment of the invention similar to that in Fig. 2, in which the strip attachment points are altered.
- Fig. 6: shows a further embodiment of the invention similar to that in Fig. 5, in which the strip attachment points have an alternative configuration.
- Fig. 7: shows a still further embodiment of the invention similar to that in Fig. 5, in which the opposite surfaces of the second and release strips are attached to the first strip.
- Fig. 8: shows a cross-sectional view of a strip for use with the invention, which is constituted by a composite structure.
- Fig. 9: shows a cross-sectional view of a composite strip comprising three sub-strips in series.

In the figures, the cross-sectional views are not drawn to scale for reasons of clarity and thus many parts thereof would in fact be relatively far thinner in practice and thus, at least as far as concerns the thickness of the arrangement shown, would be closer together. In particular, the thickness of the sheets and of the adhesive has been exaggerated.

### Detailed description of preferred embodiments:

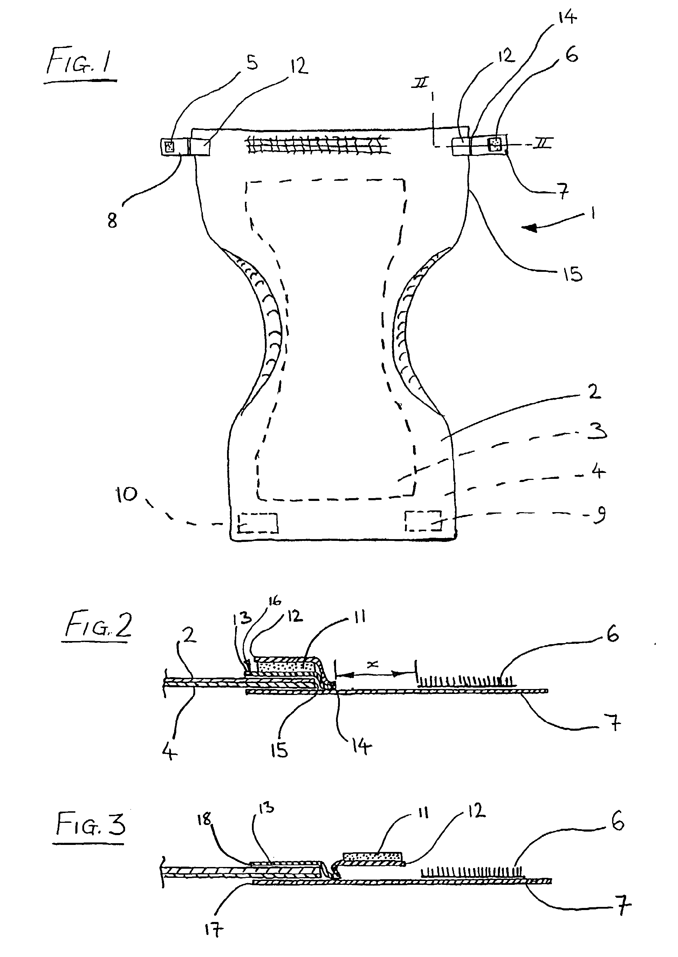

The absorbent product 1 in Figure 1 comprises a top sheet 2 providing all of, or the majority of, one surface of the product. Below the top sheet is an absorbent core 3 shown in dashed lines. On the opposite side of the product 1 there is a back sheet 4 (see also Fig. 2) which in the embodiment shown has generally the same shape and dimensions as the top sheet 2. The back sheet 4 and the top sheet 2 are preferably attached to each other at the respective peripheries. However either the top sheet 2 or the back sheet 4 can be smaller and thus be attached to the other sheet inside its outer periphery.

A fastening means in the form of releasable attachment part 6 of the hook and loop type is provided on a first material strip 7 on the upper right edge of the product, in a first waist region of said product. In this embodiment the waist region is elasticated. Similarly, at the upper left edge of the product a similar attachment means 5 is provided on an underlying first material strip 8. Each of said attachment parts 5, 6 is constituted by a hook part of a hook and loop attachment means.

On the opposite end of the article, in a second waist region, are the corresponding attachment parts 9 and 10 of the hook and loop attachment means, these being shown in dashed lines. The parts 9 and 10 will typically comprise loop material and may be in the form of two separate strips (as shown) or they may be combined as one or more longer strips extending between the longitudinal edges of the product at the second waist region. The parts 9 and 10 are preferably fixed to the back sheet 4.

Although parts 5 and 6 are illustrated as being hook parts, they could equally be loop parts. In such a case, the corresponding attachment surface constituted by parts 9 and 10 would need to be altered correspondingly to allow attachment.

The attachment means 5, 6, 9 and 10 as described above are conventional. Thus, fitting and fastening of the product using these means will be understood by the skilled man without further description thereof.

As best shown in Figures 2 and 3, the reclosure means is constituted by an adhesive 11 applied on to a second material strip 12. Preferably the adhesive will be a pressure-sensitive adhesive, although other forms of adhesive can be used.

Between the lower surface of the adhesive and the top sheet of the absorbent product there is a further strip of material which is a strip of release material 13, sometimes referred to as a release liner. The release strip 13 may be covered with a release material such as silicon on the upper surface thereof to assist removal of second strip 12 adhered thereto by means of the adhesive 11.

The strips 12 and 13 are preferably each attached to the first strip 7 at an attachment location 14. The attachment location is suitably positioned directly adjacent to the longitudinal edge 15 of the absorbent product sheet 2 and 4. Such attachment at location 14 is a fixed attachment, preferably formed by means of ultrasonic welding or hot-melt adhesive or the like.

Similarly however, the strip 13 need not in fact be attached to the first strip 7 but may instead simply be placed on the top sheet 2 at the location underneath where the adhesive 11 on strip 12 will overlie the top sheet 2, and thus its outermost end (i.e. to the right in Fig. 2) may finish proximate the edge 15 for example, rather than being attached to strip 7.

However it is preferred that the strip 13 is attached to both the top sheet 2 and to the material strip 7 since this arrangement provides a more stable and more easily usable structure. Additionally, when the strip 12 is peeled away from strip 13, the opening operation is simplified since the user does not need to hold down the free end 16 of the strip 13 in order to peel second strip 12 away from release strip 13. Indeed, whilst a free end 16 has been shown on strip 13 extending inwardly with respect to strip 12, this can be entirely dispensed with when the strip 13 is fixedly attached to the top sheet 2. However, when strip 13 is not attached to the sheet 2 and/or 4 a slight edge must be provided to allow a user to hold the release strip against the upper sheet (top sheet 2 in this case) whilst peeling the second strip 12 away therefrom.

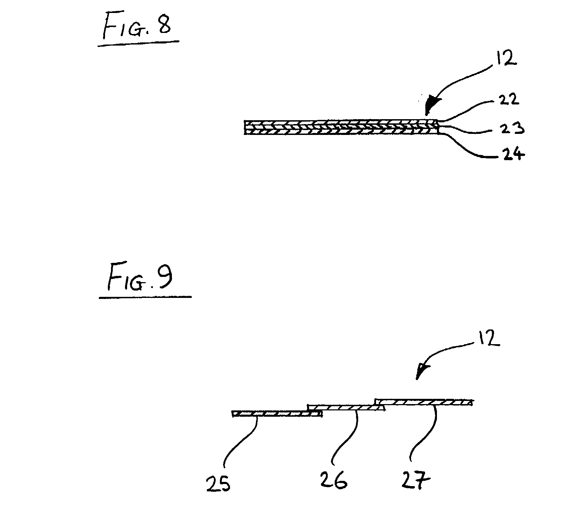

It should also be noted that although the outer end edge of strip 12 and strip 13 have been shown to be substantially coterminous in Figs. 2 to 4 with each strip attached to the strip 7 at a single location (e.g. by welding or gluing at the same or substantially the same point), each of the strips 12 and 13 may be individually attached to strip 7, the outer end edge 19 of second strip 12 thereby being attached at a location lying further outwardly (i.e. closer to hook strip 6) than the outer end edge 20 of strip 13. Such an embodiment is shown in Fig. 5. However, the distance between the outer end edges of the strips 12 and 13 in such a case is preferably kept very small, typically of the order of 0.5 mm to 4 mm, to maintain a relatively common attachment location.

Additionally, further embodiments of attachment can be employed for strips 12 and 13, such as shown in Fig. 6 in which the second strip 12 is attached to the release strip 13 at a location 21 inwards of the outer end edge 20 of the strip 13 and then attaching only strip 13 directly to the strip 7.

Further, as a further alternative to the strip lay-up as shown in Figures 2 to 6, the strips 12 and 13 could be attached to the strip 7 on their opposite surfaces as depicted in Fig. 7, by first curving the outer ends of the strips 12 and 13 back on themselves so that, at their end portions, their opposite surfaces will face the strip 7. In so doing, a portion of the second strip 12 can be made to underlie part of the curved edge portion of strip 13. This has the advantage that the outwardly-facing, curved edge portion of inner strip 13 will also tend to force the strip 12 outwards (i.e. to the right in the Figure), which will assist in preventing the strip 12 from re-assuming a closed position once it has been folded opened for use; the dashed lines in Fig. 6 indicating the opened position.

In terms of the resistance of the strip 13 against bending forces it is preferred if the end 18 of strip 13 lies approximately in lateral alignment with the end 17 of strip 7, or further inwardly thereof.

With such an arrangement the flexibility of strip 7 to the right of attachment location 14 is far greater than the combination of layers 7, 2, 4, 13, 11 and 12, and therefore provides a natural hinge line at location 14 which means that virtually all flexing of the tab 7 during fitting is done outwardly of the attachment point 14.

The distance "x" (i.e. the distance between the location 14 and the hook tab 6 inner edge) as shown in the Figures may be varied, but typically will be of the order of 1 cm to 4 cm. However different dimensions may be suitable. Similarly, whilst the arrangement shown in Fig. 3 (where the adhesive 11 is exposed for use) depicts the strip 12 not extending the complete distance "x", and thus stopping short of the hook attachment part 6, the distance "x" may however be shorter than the length of the strip 12 such that the adhesive will overlie at least part, if not all, of the hook part 6.

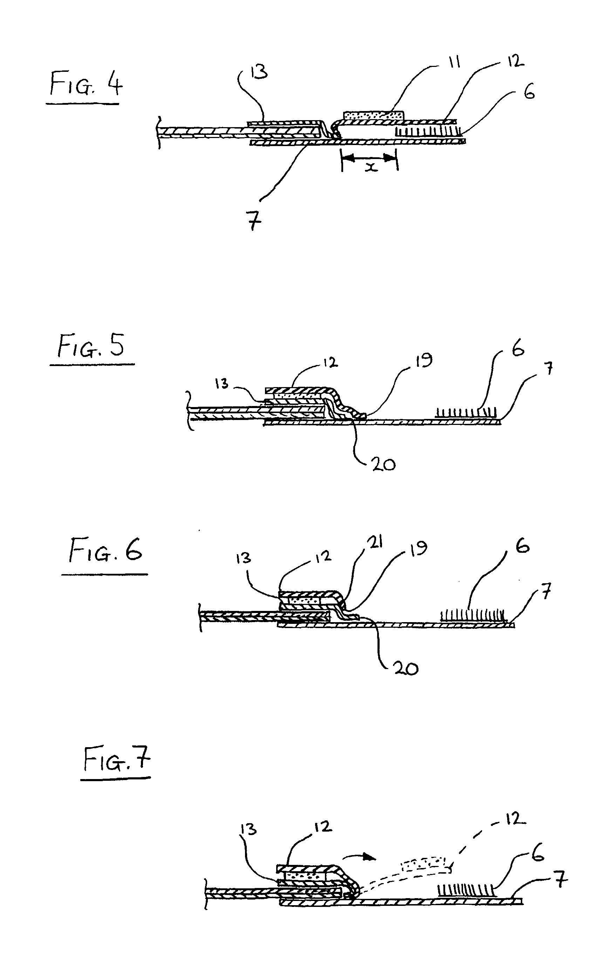

Such an arrangement is shown in Fig. 4. An advantage is thus present when the distance "x" is shorter than the second strip 12, since this will then (at least to some extent) prevent the hook elements on hook part 6 catching on the user's clothes (e.g. cuffs of a pullover for example) or other items, when the user is proceeding to roll up the product for reclosure. The adhesive 11 in Fig. 4 is shown as extending only part way along strip 12 and this is all that is required to provide the reclosure means. The remainder of the strip 12 which is not coated with adhesive 11 provides a non-adhesive barrier against contact with the hook means during rolling up. Even when contact is however made with the pressure sensitive adhesive 11, this will be less troublesome than contact with the hooks of hook part 6 since very little pressure would normally be applied when the user's clothes touch the adhesive during the reclosure procedure, such that the pressure sensitive adhesive effect of the adhesive 11 is not activated.

In other cases it may be suitable to make "X" larger such that the outer end of unfolded strip 12 will not overlie the hook strip 6.

Additionally, where the strip 12 is longer than the strip 13 and the adhesive 11 coating, an edge is provided which is not attached and can thus be grasped between the index finger and thumb for peeling away from release strip 13.

To obtain the configuration shown in Fig. 3 in which the adhesive 11 is exposed, the user simply needs to peel up the second strip 12 with adhesive 11 coated thereon, away from release strip 13. Initial access to the edge of the strip can be gained with either a fingernail or the side of a finger if there is little or no overlap between the strip 12 and the adhesive 11. As described above however, if there is a small excess of material of second strip 12 extending inwardly beyond the adhesive 11 (i.e. to the left in Fig. 2), this can either be gripped or accessed with a finger for example.

As will be apparent from the arrangement of the reclosure means, the attachment force between the adhesive 11 and release strip 13 need only be very small for acceptable and reliable functioning. When the strip 13 is attached to the top sheet 2 or back sheet 4, the attachment force between the strip 13 and the respective sheet 2 or 4 should however be greater than the adhesive force between the adhesive 11 and the strip 13. In this way, when the second strip 12 is peeled away from the release strip 13, the release strip will stay firmly attached to the top sheet 2.

Although the invention has been described above with reference to several preferred embodiments thereof, the scope of the invention is not limited thereto but is instead defined by the full scope of the appended claims.

For example, although the strip 13 has been depicted as overlying or being attached to the top sheet 2, the periphery of the top sheet 2 may for example lie inwardly of the inner edge 18 of the release strip 13, such that the release strip then overlies and is attached to the upper surface of the back sheet. Similarly, the back sheet may have a periphery lying inside that of the top sheet, such that connection to the top sheet would occur. Where one of the sheets 2 or 4 lies only a very small distance inside the other of said sheets, the strip 13 may of course be attached to both of said sheets.

Similarly it will be appreciated that whilst the invention has been described with respect to an absorbent garment having reclosure means on both the left and right sides, only one reclosure means is actually necessary to ensure adequate functioning.

The material and construction of the strips 7, 12 and 13 can be varied within wide limits. For example, strip 12 itself might be constituted by a laminated structure, or a composite structure such as shown in Fig. 8 in which two outer layers 22 and 24 of e.g. nonwoven material sandwich therebetween a layer 23 of polymer film (such as PP or PE for example). Clearly only two or more than three layers can be used to form a laminated or composite strip for use with the invention.

However, it should be understood that the actual materials used for the strips are not limiting for the invention, as long as the strips can fulfil their intended purpose.

A further possibility for the structure of any of the strips is that the strip(s) may comprise several sub-strip elements which are added together in series (i.e. from one end of the strip to another), such that each sub-strip is attached to, and overlaps, an underlying sub-strip by a small distance. Attachment may typically be by means of adhesive or welding. Such a structure may be usefully employed for strip 12 for example, as shown in Fig. 9. In this embodiment, strip 12 is formed of three sub-strips 25, 26 and 27 respectively. Two or more than three sub-strips may also be used of course, depending on the application. The sub-strip 25 could be the sub-strip which is to be attached at location 14 to strip 7 for example (or to strip 13, as described in other embodiments above). In such a case, sub-strip 25 could comprise, or consist of, a very flexible material so as to aid the hinging of the strip 12 around the attachment location 14 (see e.g. Fig. 2). The materials of sub-strips 26 and 27 can then have other specific properties. For example, sub-strip 26 could be made particularly suitable for retaining the adhesive 11 whilst sub-strip 27 could present a high-friction or patterned surface allowing easy grasping thereof for opening the strip 12. In a similar manner, the material of the sub-strip 25 may have the property that once the hinge is folded open, it substantially prevents the strip 12 from moving back to its closed position by using a material with minimal elastic memory.

In a further embodiment (not shown), the sub-strip combination may comprise three or more sub-strips in which the middle one of each three sub-strips overlies both the adjacent strips. Thus, when considering the embodiment of Fig. 9 for example, the strip 26 would overly the strip 27 and not only sub-strip 25. This of course may be advantageous where it is desired to have a larger surface area exposed on sub-strip 26, without lengthening the strip 12.

Further lay-ups of the sub-strips, laminates or composites will be readily understood by the skilled person upon reading the aforegoing and are intended to be encompassed within the scope of the invention.

## Claims

1. Absorbent product (1) for absorbing bodily exudate, said absorbent product (1) comprising at least a back sheet (4), a top sheet (2) and an attachment means (5, 8, 10; 6, 7, 9), said attachment means including one attachment part (5, 6) of a hook and loop fastening means, said one attachment part (5, 6) being positioned on a first material strip (7) attached on one side of said absorbent product at an edge (15) thereof such that said one attachment part (5, 6) lies outwardly of said edge (15), wherein said attachment means further comprises an adhesive reclosure means, **characterized in that** the adhesive (11) of said reclosure means is attached to a second material strip (12) inwardly overlying said edge (15) of one of said sheets (2, 4) of said absorbent article, and in that a strip of release material (13) is interposed between said one (2, 4) of said sheets and said adhesive (11).

2. Absorbent product according to claim 1, **characterized in that** said strip of release material (13) is attached to said one (2, 4) of said sheets of said absorbent product (1).

3. Absorbent product according to claim 2, **characterized in that** the adhesive force between the strip of release material (13) and the adhesive (11) of said reclosure means is less than the attachment force between said one (2, 4) of said sheets and said strip of release material (13).

4. Absorbent product according to any one of the preceding claims, **characterized in that** said one of said sheets (2, 4) is said top sheet (2).

5. Absorbent product according to any one of the preceding claims, **characterized in that** one end of said second strip of material and one end of said release material strip are attached to said first material strip at a location (14) laterally outwardly of and immediately adjacent the edge (15) of said absorbent product (1).

6. Absorbent product according to any one of the preceding claims, **characterized in that** said absorbent product edge is formed by a longitudinal edge (15) of the top sheet (2) and/or back sheet (4) of said absorbent product (1).

7. Absorbent product according to any one of the preceding claims, **characterized in that** said second material strip (12) is attached to said first material strip at an attachment location (14), and in that said first attachment part (6) is positioned at a separation distance (x) from said attachment location (14), and in that the length of said second strip (12) from said attachment location (14) to its outer end is greater than said separation distance (x).

8. Absorbent product according to any one of the preceding claims, **characterized in that** said adhesive (11) is a pressure-sensitive adhesive compatible with at least the material of said back sheet (4).

9. Absorbent product according to any one of the preceding claims, **characterized in that** said one attachment part (5, 6) is a strip of hook attachment material.

10. Absorbent product (1) according to any one of the preceding claims, **characterized in that** said absorbent product is a disposable absorbent product.
